# EUROPEAN PATENT SPECIFICATION

(11) **EP 4 571 640 B1**
(45) Date of publication and mention of the grant of the patent: **29.07.2026**
(21) Application number: 24218542.9
(22) Date of filing: 10.12.2024
(51) Int. Cl.: G06T 7/00, G06T 7/11

(54) **METHOD FOR QUANTIFYING BREAST COMPOSITION**
VERFAHREN ZUR QUANTIFIZIERUNG EINER BRUSTZUSAMMENSETZUNG
PROCÉDÉ DE QUANTIFICATION DE COMPOSITION MAMMAIRE

(30) Priority: 13.12.2023 KR 20230180626
(43) Date of publication of application: 18.06.2025
(73) Proprietor: BeamWorks Inc., Daegu 41404 (KR)
(72) Inventor: Kim, Jaeil, 41584 Daegu (KR); Kim, Won Hwa, 42119 Daegu (KR); Park, Sungjoon, 41416 Daegu (KR); Moon, Woo Kyung, 06288 Seoul (KR)
(74) Representative: Petraz, Gilberto Luigi

(56) References cited:
- WO-A1-2023/200990
- US-A1- 2020 372 637
- MOGHBEL MEHRDAD ET AL: "A review of breast boundary and pectoral muscle segmentation methods in computer-aided detection/diagnosis of breast mammography", ARTIFICIAL INTELLIGENCE REVIEW, SPRINGER NETHERLANDS, NL, vol. 53, no. 3, 29 May 2019 (2019-05-29), pages 1873 - 1918, XP037063541, ISSN: 0269-2821, [retrieved on 20190529], DOI: 10.1007/S10462-019-09721-8

## Description

### CROSS-REFERENCE TO RELATED APPLICATIONS

This application claims priority to and the benefit of Korean Patent Application No. 10-2023-0180626 filed in the Korean Intellectual Property Office on 13 December 2023.

### TECHNICAL FIELD

The present disclosure relates to a method for quantifying a breast composition, and more particularly, to a technology of quantifying a breast tissue component or a glandular tissue component using a breast ultrasound image, as well as a computer program stored in a non-transitory computer-readable storage medium and a computing device.

### BACKGROUND ART

A tissue of a breast is composed at a ratio of a fibro-glandular tissue (FGT) and a fat. A breast tissue composition is related to a detection sensitivity and an occurrence risk of breast cancer, so software is required for quantitative analysis. If a reader subjectively performs quantification, there is a low quantification match between the readers, and in particular, there is a problem that it is more uncertain when various readers perform quantification.

Further, in order to analyze the breast composition, medical imaging techniques such as breast angiography and ultrasound can be used. There is a limit that dense breasts in the breast angiography are difficult to distinguish between breast tissues having a risk. On the other hand, the ultrasound can distinguish a glandular tissue component, and is widely used for diagnosis inspection due to advantages such as low cost, use accessibility, and safety without radiation exposure. However, a boundary between ultrasound tissues is similar, so it is difficult to distinguish the boundary, and a resolution is lowered due to an image quality, shadow and noise that depend on user skills. Accordingly, quantification analysis software using the ultrasound is required.
Korean Patent Unexamined Publication No. 10-2014-0109320 (September 15, 2014) discloses a method and an apparatus for ultrasound image processing and apparatus for diagnosing breast cancer. US 2020/372637 discloses a method for quantifying a breast composition performed by a computing device as of the preamble of claim 1. WO 2023/200990 and MOGHBEL MEHRDAD ET AL: "A review of breast boundary and pectoral muscle segmentation methods in computer-aided detection/diagnosis of breast mammography", ARTIFICIAL INTELLIGENCE REVIEW, SPRINGER NETHERLANDS, NL, vol. 53, no. 3, 29 May 2019 (2019-05-29), pages 1873-1918, (XP037063541) disclose known methods for quantifying a breast composition performed by a computing device.

### SUMMARY OF THE INVENTION

The present invention, which is identified in the appended set of claims, has been made in an effort to provide a method for quantifying a breast composition, which can quantitatively measure at least one of a breast tissue component or a glandular tissue component automatically from breast ultrasound, and classify a grade by using deep learning technology.

Meanwhile, a technical object to be achieved by the present disclosure is not limited to the above-mentioned technical object, and various technical objects can be included within the scope which is apparent to those skilled in the art from contents to be described below.

The present invention provides a method for quantifying a breast composition performed by a computing device, the method further including: acquiring an ultrasound image; identifying a breast region in the ultrasound image; identifying a fibro-glandular tissue region in the ultrasound image, comprising generating at least one of a fibro-glandular inference region mask and a fibro-glandular region binary map using a deep learning model; and quantifying at least one of a breast tissue component or a glandular tissue component based on the identified breast region and the identified fibro-glandular tissue region.

The identifying of the breast region in the ultrasound image includes generating a breast inference region mask in the ultrasound image using a deep learning model, generating a breast region binary map based on the generated breast inference region mask; and generating a breast region division mask based on the breast region binary map.

The breast region division mask is acquired based on a largest region among regions included in the breast region binary map.

The identifying of the fibro-glandular tissue region in the ultrasound image includes generating a fibro-glandular inference region mask in the ultrasound image using a deep learning model, generating a fibro-glandular region binary map based on the fibro-glandular inference region mask, and generating a fibro-glandular region division mask based on the fibro-glandular region binary map.

In an exemplary embodiment, the breast inference region mask may include a breast region probability map, and the generating of the breast region binary map based on the generated breast inference region mask may include generating the breast region binary map by classifying the breast region probability map based on a predetermined threshold.

In an exemplary embodiment, the fibro-glandular inference region mask includes a fibro-glandular region probability map, and the generating of the fibro-glandular region binary map based on the generated fibro-glandular inference region mask may include generating the fibro-glandular region binary map by classifying the fibro-glandular region probability map based on a predetermined threshold.

In an exemplary embodiment, the generating of the fibro-glandular region division mask based on the fibro-glandular region binary map may include generating an in-breast fibro-glandular region mask by performing an operation for the breast region division mask and the fibro-glandular region binary map, and generating the fibro-glandular region division mask based on the in-breast fibro-glandular region mask.

In an exemplary embodiment, the fibro-glandular region division mask may be acquired based on a largest region among regions included in the in-breast fibro-glandular region mask.

In an exemplary embodiment, the quantifying of at least one of the breast tissue component or the glandular tissue component based on the identified breast region and the identified fibro-glandular tissue region may include identifying a glandular tissue region based on the identified fibro-glandular tissue region, and quantifying the glandular tissue component by analyzing the identified fibro-glandular tissue region and the identified glandular tissue region.

In an exemplary embodiment, the identifying of the glandular tissue region based on the identified fibro-glandular tissue region may include generating a glandular tissue region binary map based on the fibro-glandular region division mask, and generating a glandular tissue region division mask based on the glandular tissue region binary map.

In an exemplary embodiment, the generating of the glandular tissue region binary map based on the fibro-glandular region division mask may include extracting a glandular tissue region of interest mask from the fibro-glandular region division mask, detecting a polygonal type region of interest including the glandular tissue region of interest, performing histogram based normalization for the ultrasound image inside the polygonal type region of interest, generating a region of interest normalization ultrasound image by using the ultrasound image inside the polygonal type region of interest in which the histogram based normalization is performed and an ultrasound image outside the polygonal type region of interest, generating a glandular tissue region of interest image by performing an operation for the region of interest normalization ultrasound image and the glandular tissue region of interest mask, and generating the glandular tissue region binary map by classifying the glandular tissue region of interest image based on a predetermined threshold.

In an exemplary embodiment, the quantifying of at least one of the breast tissue component or the glandular tissue component based on the identified breast region and the identified fibro-glandular tissue region may include computing a ratio of the breast tissue component by using a pixel value of the fibro-glandular region division mask and a pixel value of the breast region division mask, and mapping the ratio of the breast tissue component according to a breast tissue component (BTC) grade.

In an exemplary embodiment, the quantifying of at least one of the breast tissue component or the glandular tissue component based on the identified breast region and the identified fibro-glandular tissue region may include computing a ratio of the glandular tissue component by using a pixel value of the glandular tissue region division mask and the pixel value of the fibro-glandular region division mask, and mapping the ratio of the glandular tissue component according to a glandular tissue component (GTC) grade.

According to another aspect, the present invention provides a computer-readable storage medium comprising a computer program. When the computer program is executed by one or more processors, the computer program allows the one or more processors to perform the operations for quantifying a breast composition as indicated above.

In an exemplary embodiment, the operation of identifying the breast region in the ultrasound image includes an operation of generating a breast inference region mask in the ultrasound image using a deep learning model, an operation of generating a breast region binary map based on the generated breast inference region mask, and an operation of generating a breast region division mask based on the breast region binary map.

In an exemplary embodiment, the breast inference region mask may include a breast region probability map, and the operation of generating the breast region binary map based on the generated breast inference region mask may include an operation generating the breast region binary map by classifying the breast region probability map based on a predetermined threshold.

In an exemplary embodiment, the operation of identifying the fibro-glandular tissue region in the ultrasound image may include an operation of generating a fibro-glandular inference region mask in the ultrasound image using a deep learning model, an operation of generating a fibro-glandular region binary map based on the fibro-glandular inference region mask, and an operation of generating a fibro-glandular region division mask based on the fibro-glandular region binary map.

In an exemplary embodiment, the fibro-glandular inference region mask includes a fibro-glandular region probability map, and the operation of generating the fibro-glandular region binary map based on the generated fibro-glandular inference region mask may include an operation of generating the fibro-glandular region binary map by classifying the fibro-glandular region probability map based on a predetermined threshold.

In an exemplary embodiment, the operation of quantifying of at least one of the breast tissue component or the glandular tissue component based on the identified breast region and the identified fibro-glandular tissue region may include an operation of identifying a glandular tissue region based on the identified fibro-glandular tissue region, and an operation of quantifying the glandular tissue component by analyzing the identified fibro-glandular tissue region and the identified glandular tissue region.

**In** an exemplary embodiment, the operation of quantifying at least one of the breast tissue component or the glandular tissue component based on the identified breast region and the identified fibro-glandular tissue region may include an operation of computing a ratio of the breast tissue component by using a pixel value of the fibro-glandular region division mask and a pixel value of the breast region division mask, and an operation of mapping the ratio of the breast tissue component according to a breast tissue component (BTC) grade.

**In** an exemplary embodiment, the operation of quantifying at least one of the breast tissue component or the glandular tissue component based on the identified breast region and the identified fibro-glandular tissue region may include an operation of computing a ratio of the glandular tissue component by using a pixel value of the glandular tissue region division mask and the pixel value of the fibro-glandular region division mask, and an operation of mapping the ratio of the glandular tissue component according to a glandular tissue component (GTC) grade.

Yet according to another aspect, the present invention provides a computing device. The device includes: at least one processor; and a memory, and the at least one processor is configured to acquire an ultrasound image, identify a breast region in the ultrasound image, identify a fibro-glandular tissue region in the ultrasound image, comprising generating at least one of a fibro-glandular inference region mask and a fibro-glandular region binary map using a deep learning model, and quantify at least one of a breast tissue component or a glandular tissue component based on the identified breast region and the identified fibro-glandular tissue region.

The identifying of the breast region in the ultrasound image includes generating a breast inference region mask in the ultrasound image using a deep learning model, generating a breast region binary map based on the generated breast inference region mask; and generating a breast region division mask based on the breast region binary map.

The breast region division mask is acquired based on a largest region among regions included in the breast region binary map.

The identifying of the fibro-glandular tissue region in the ultrasound image includes generating a fibro-glandular inference region mask in the ultrasound image using a deep learning model, generating a fibro-glandular region binary map based on the fibro-glandular inference region mask, and generating a fibro-glandular region division mask based on the fibro-glandular region binary map.

In an exemplary embodiment, the at least one processor is configured to generate a breast inference region mask in the ultrasound image using a deep learning model, generate a breast region binary map based on the generated breast inference region mask, and generate a breast region division mask based on the breast region binary map.

In an exemplary embodiment, the breast inference region mask may include a breast region probability map, and the at least one processor may be configured to generate the breast region binary map by classifying the breast region probability map based on a predetermined threshold.

In an exemplary embodiment, the at least one processor is configured to generate a fibro-glandular inference region mask in the ultrasound image using a deep learning model, generate a fibro-glandular region binary map based on the generated fibro-glandular inference region mask, and generate a fibro-glandular region division mask based on the fibro-glandular region binary map.

In an exemplary embodiment, the fibro-glandular inference region mask may include a fibro-glandular region probability map, and the at least one processor may be configured to generate the fibro-glandular region binary map by classifying the fibro-glandular region probability map based on a predetermined threshold.

In an exemplary embodiment, the at least one processor may be configured to identifying a glandular tissue region based on the identified fibro-glandular tissue region, and quantify the glandular tissue component by analyzing the identified fibro-glandular tissue region and the identified glandular tissue region.

In an exemplary embodiment, the least one processor may be configured to compute a ratio of the breast tissue component by using a pixel value of the fibro-glandular region division mask and a pixel value of the breast region division mask, and mapping the ratio of the breast tissue component according to a breast tissue component (BTC) grade.

In an exemplary embodiment, the at least one processor may be configured to compute a ratio of the glandular tissue component by using a pixel value of a glandular tissue region division mask and the pixel value the fibro-glandular region division mask, and map the ratio of the glandular tissue component according to a glandular tissue component (GTC) grade.

According to the present disclosure, at least one of a breast tissue component or a glandular tissue component is automatically quantitatively measured, and a grade is classified by using deep learning technology to assist breast tissue analysis of a reader in diagnosis of a breast ultrasound, and increase a matching degree between the readers, and a matching degree in the reader.

Meanwhile, the effects of the present disclosure are not limited to the above-mentioned effects, and various effects can be included within the scope which is apparent to those skilled in the art from contents to be described below.

### BRIEF DESCRIPTION OF THE DRAWINGS

FIG. 1 is a block diagram of a computing device for quantifying a breast composition according to an exemplary embodiment of the present disclosure.
FIG. 2 is a conceptual diagram illustrating a neural network according to an exemplary embodiment of the present disclosure.
FIG. 3 is a block diagram of a plurality of modules for quantifying a breast composition according to an exemplary embodiment of the present disclosure.
FIG. 4 is a diagram schematically illustrating an operation for quantifying a breast composition according to an exemplary embodiment of the present disclosure.
FIG. 5A is a diagram for describing an operation of computing a ratio and a degree of a breast tissue component according to an exemplary embodiment of the present disclosure.
FIG. 5B is a diagram for describing an operation of computing a ratio and a degree of a glandular tissue component according to an exemplary embodiment of the present disclosure.
FIG. 6 is a flowchart illustrating a method for quantifying a breast component according to an exemplary embodiment of the present disclosure.
FIG. 7 is a simple and normal schematic view of an exemplary computing environment in which the exemplary embodiments of the present disclosure may be implemented.

### DETAILED DESCRIPTION

Various exemplary embodiments will now be described with reference to drawings. In the present specification, various descriptions are presented to provide appreciation of the present disclosure. However, it is apparent that the exemplary embodiments can be executed without the specific description.

"Component", "module", "system", and the like which are terms used in the specification refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented exemplary embodiments is provided so that those skilled in the art of the present disclosure use or implement the present disclosure. Various modifications to the exemplary embodiments will be apparent to those skilled in the art. Generic principles defined herein may be applied to other embodiments without departing from the scope of the present disclosure. Therefore, the present disclosure is not limited to the exemplary embodiments presented herein. The present disclosure should be analyzed within the widest range which is coherent with the principles and new features presented herein.

In the present disclosure, a network function and an artificial neural network and a neural network may be interchangeably used.

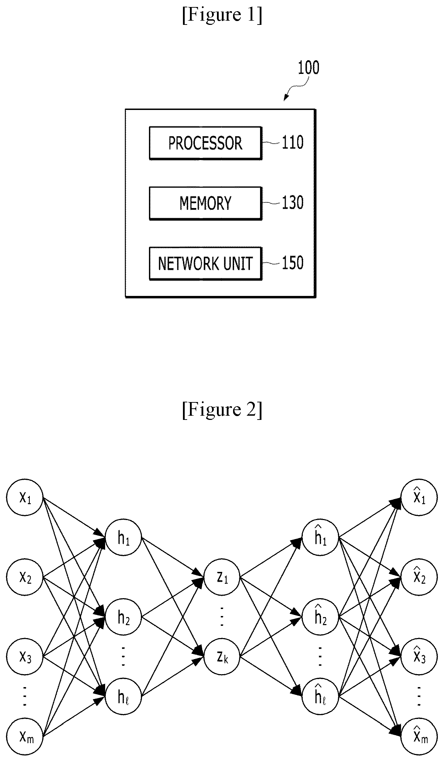

FIG. 1 is a block diagram of a computing device for quantifying a breast composition according to an exemplary embodiment of the present disclosure.

A configuration of the computing device 100 illustrated in FIG. 1 is only an example shown through simplification. In an exemplary embodiment of the present disclosure, the computing device 100 may include other components for performing a computing environment of the computing device 100 and only some of the disclosed components may constitute the computing device 100.

The computing device 100 includes a processor 110, a memory 130, and possibly a network unit 150.

The processor 110 may be constituted by one or more cores and may include processors for data analysis and deep learning, which include a central processing unit (CPU), a general purpose graphics processing unit (GPGPU), a tensor processing unit (TPU), and the like of the computing device. The processor 110 may read a computer program stored in the memory 130 to perform data processing for machine learning according to an exemplary embodiment of the present disclosure. According to an exemplary embodiment of the present disclosure, the processor 110 may perform a calculation for training the neural network. The processor 110 may perform calculations for training the neural network, which include processing of input data for training in deep learning (DL), extracting a feature in the input data, calculating an error, updating a weight of the neural network using backpropagation, and the like. At least one of the CPU, GPGPU, and TPU of the processor 110 may process training of a network function. For example, both the CPU and the GPGPU may process the training of the network function and data classification using the network function. Further, in an exemplary embodiment of the present disclosure, processors of a plurality of computing devices may be used together to process the training of the network function and the data classification using the network function. Further, the computer program executed in the computing device according to an exemplary embodiment of the present disclosure may be a CPU, GPGPU, or TPU executable program.

According to an exemplary embodiment of the present disclosure, the memory 130 may store any type of information generated or determined by the processor 110 and any type of information received by the network unit 150.

According to an exemplary embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

The network unit 150 according to an exemplary embodiment of the present disclosure may use various wired communication systems such as public switched telephone network (PSTN), x digital subscriber line (xDSL), rate adaptive DSL (RADSL), multi rate DSL (MDSL), very high speed DSL (VDSL), universal asymmetric DSL (UADSL), high bit rate DSL (HDSL), and local area network (LAN).

The network unit 150 presented in the present disclosure may use various wireless communication systems such as code division multi access (CDMA), time division multi access (TDMA), frequency division multi access (FDMA), orthogonal frequency division multi access (OFDMA), single carrier-FDMA (SC-FDMA), and other systems.

In the present disclosure, the network unit 150 may be configured regardless of a communication aspect, such as wired communication and wireless communication, and may be configured by various communication networks, such as a Personal Area Network (PAN) and a Wide Area Network (WAN). Further, the network may be a publicly known World Wide Web (WWW), and may also use a wireless transmission technology used in short range communication, such as Infrared Data Association (IrDA) or Bluetooth.

FIG. 2 is a conceptual diagram illustrating a neural network according to an exemplary embodiment of the present disclosure.

Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, a definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

In the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, generative adversarial networks (GAN), a restricted Boltzmann machine (RBM), a deep belief network (DBN), a Q network, a U network, a Siam network, a Generative Adversarial Network (GAN), and the like. The description of the deep neural network described above is just an example and the present disclosure is not limited thereto.

In an exemplary embodiment of the present disclosure, the network function may include the auto encoder. The auto encoder may be a kind of artificial neural network for outputting output data similar to input data. The auto encoder may include at least one hidden layer and odd hidden layers may be disposed between the input and output layers. The number of nodes in each layer may be reduced from the number of nodes in the input layer to an intermediate layer called a bottleneck layer (encoding), and then expanded symmetrical to reduction to the output layer (symmetrical to the input layer) in the bottleneck layer. The auto encoder may perform non-linear dimensional reduction. The number of input and output layers may correspond to a dimension after preprocessing the input data. The auto encoder structure may have a structure in which the number of nodes in the hidden layer included in the encoder decreases as a distance from the input layer increases. When the number of nodes in the bottleneck layer (a layer having a smallest number of nodes positioned between an encoder and a decoder) is too small, a sufficient amount of information may not be delivered, and as a result, the number of nodes in the bottleneck layer may be maintained to be a specific number or more (e.g., half of the input layers or more).

The neural network may be trained in at least one scheme of supervised learning, unsupervised learning, semi supervised learning, or reinforcement learning. The learning of the neural network may be a process in which the neural network applies knowledge for performing a specific operation to the neural network.

The neural network may be trained in a direction to minimize errors of an output. The training of the neural network is a process of repeatedly inputting training data into the neural network and calculating the output of the neural network for the training data and the error of a target and back-propagating the errors of the neural network from the output layer of the neural network toward the input layer in a direction to reduce the errors to update the weight of each node of the neural network. In the case of the supervised learning, the training data labeled with a correct answer is used for each training data (i.e., the labeled training data) and in the case of the unsupervised learning, the correct answer may not be labeled in each training data. That is, for example, the training data in the case of the supervised learning related to the data classification may be data in which category is labeled in each training data. The labeled training data is input to the neural network, and the error may be calculated by comparing the output (category) of the neural network with the label of the training data. As another example, in the case of the unsupervised learning related to the data classification, the training data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation. A variation amount of the updated connection weight of each node may be determined according to a learning rate. Calculation of the neural network for the input data and the back-propagation of the error may constitute a training cycle (epoch). The learning rate may be applied differently according to the number of repetition times of the training cycle of the neural network. For example, in an initial stage of the training of the neural network, the neural network ensures a certain level of performance quickly by using a high learning rate, thereby increasing efficiency and uses a low learning rate in a latter stage of the training, thereby increasing accuracy.

In training of the neural network, the training data may be generally a subset of actual data (i.e., data to be processed using the trained neural network), and as a result, there may be a training cycle in which errors for the training data decrease, but the errors for the actual data increase. Overfitting is a phenomenon in which the errors for the actual data increase due to excessive training of the training data. For example, a phenomenon in which the neural network that trains a cat by showing a yellow cat sees a cat other than the yellow cat and does not recognize the corresponding cat as the cat may be a kind of overfitting. The overfitting may act as a cause which increases the error of the machine learning algorithm. Various optimization methods may be used in order to prevent the overfitting. In order to prevent the overfitting, a method such as increasing the training data, regularization, dropout of omitting a part of the node of the network in the process of training, utilization of a batch normalization layer, etc., may be applied.

The present invention relates to a method of performing a breast composition evaluation technique of breast ultrasound, and quantification of a breast tissue component and a glandular tissue component, and grade classification by using deep learning technology from a breast ultrasound image. Further, the present invention is a technology for software which performs automatic quantification and grade classification of a breast tissue component and a glandular tissue component in ultrasound.

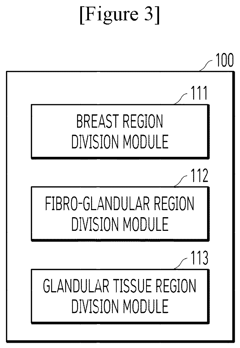

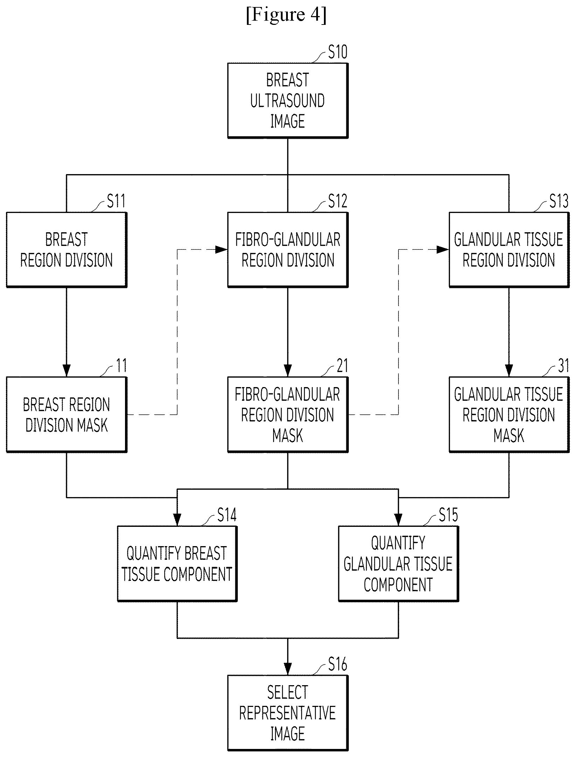

FIG. 3 is a block diagram of a plurality of modules for quantifying a breast composition according to an exemplary embodiment of the present disclosure and FIG. 4 is a diagram schematically illustrating an operation for quantifying a breast composition according to an exemplary embodiment of the present disclosure.

According to an exemplary embodiment of the present disclosure, the computing device 100 may include a breast region division module 111, a fibro-glandular region division module 112, and a glandular tissue region division module 113. Meanwhile, multiple modules that may be included in the computing device 100 may be controlled by the processor 110 or implemented by an operation of the processor 110. Further, in order to quantify the breast component, modules that may be included in the computing device 100 are not limited to the multiple modules described above, and may include additional modules. Hereinafter, exemplary multiple modules for quantifying the breast component will be described in more detail.

According to an exemplary embodiment of the present disclosure, the computing device 100 may acquire an ultrasound image (S10). The ultrasound image may be an ultrasound image acquired by an ultrasound probe which is equipment that receives an echo reflected after ultrasound inspection equipment generates and transmits an ultrasound wave. Further, the ultrasound image may include a medical image acquired by a freeze mode of the probe is activated. As an example, the computing device 100 may scan a breast by using the ultrasound equipment (e.g., the ultrasound probe, etc.), and acquire the ultrasound image.

According to an exemplary embodiment of the present disclosure, the breast region division module 111 may identify (infer) a breast region in the ultrasound image (S11). First, prior to identifying (inferring) the breast region, the breast region division module 111 may i) perform preprocessing of the acquired ultrasound image. The breast region division module 111 may perform preprocessing of controlling input image sizes for ultrasound images having various sizes and pixel values. Further, the breast region division module 111 may perform preprocessing of normalizing a pixel range for the ultrasound image. However, a preprocessing operation is not limited thereto, and various processing operations for application to a deep learning model may be performed.

Next, the breast region division module 111 may ii) generate a breast inference region mask in the ultrasound image by using the deep learning model. Here, the ultrasound image may be the ultrasound image preprocessed through various operations described above. As an example, the deep learning model may include U-NET, MASK R-CNN, DeepLap, PSPNet, Fully Convolutional Network (FCN), SegNet, ENet, etc., but is not limited thereto, and may include a deep learning model which is previously developed or developed afterward. For reference, the deep learning model may be a model trained to infer the breast region. In other words, the breast region division module 111 may generate a breast inference region mask in the preprocessed ultrasound image by using the deep learning model trained to inference the breast region. For example, the breast inference region mask may include a breast region probability map having a size which is the same as the input image size. A range of the probability map may include a value between 0 and 1.

Further, the breast region division module 111 may generate a breast region binary map based on the generated breast inference region mask. More specifically, the breast region division module 111 classifies the breast region probability map based on a predetermined threshold (e.g., 0.5) to generate the breast region binary map. For example, the breast region division module 111 assigns 0 when the breast region probability map is equal to or less than the threshold based on the predetermined threshold (e.g., a case where the breast region probability map corresponds to 0 to 0.5), and assigns 1 when the breast region probability map is more than the threshold (a case where the breast region probability map corresponds to 0.6 to 1) to generate the breast region binary map.

Next, the breast region division module 111 may iii) generate a breast region division mask 11 based on the breast region binary map. For example, the breast region division module 111 may generate the breast region division mask by performing an image processing task for the breast region binary map. The breast region division mask is acquired based on a largest region among regions included in the breast region binary map. The breast region division module 111 leaves only the largest region in the breast region binary map to generate the breast region division mask 11.

According to an exemplary embodiment of the present disclosure, the fibro-glandular region division module 112 may identify (infer) a fibro-glandular tissue region in the ultrasound image (S12). First, prior to identifying (inferring) the fibro-glandular tissue region, the fibro-glandular region division module 112 may i) perform preprocessing of the acquired ultrasound image. The fibro-glandular region division module 112 may perform preprocessing of controlling input image sizes for ultrasound images having various sizes and pixel values. Further, the fibro-glandular region division module 112 may perform the preprocessing of normalizing the pixel range for the ultrasound image. However, the preprocessing operation is not limited thereto, and various processing operations for application to the deep learning model may be performed.

Next, fibro-glandular region division module 112 may ii) generate a fibro-glandular inference region mask by using the deep learning model. Here, the ultrasound image may be the ultrasound image preprocessed through various operations described above. As an example, the deep learning model may include U-NET, MASK R-CNN, DeepLap, PSPNet, Fully Convolutional Network (FCN), SegNet, ENet, etc., but is not limited thereto, and may include a deep learning model which is previously developed or developed afterward. For reference, the deep learning model may be a model trained to infer a fibro-glandular region. In other words, the fibro-glandular region division module 112 may generate a fibro-glandular inference region mask in the preprocessed ultrasound image by using the deep learning model trained to inference the region. For example, the fibro-glandular inference region mask may include a fibro-glandular region probability map having a size which is the same as the input image size. A range of the probability map may include a value between 0 and 1.

Further, the fibro-glandular region division module 112 may generate a fibro-glandular region binary map based on the generated fibro-glandular inference region mask. More specifically, the fibro-glandular region division module 112 classifies the fibro-glandular region probability map based on a predetermined threshold (e.g., 0.5) to generate the fibro-glandular region binary map. For example, the fibro-glandular region division module 112 assigns 0 when the fibro-glandular region probability map is equal to or less than the threshold based on the predetermined threshold (e.g., a case where the fibro-glandular region probability map corresponds to 0 to 0.5), and assigns 1 when the fibro-glandular region probability map is more than the threshold (a case where the fibro-glandular region probability map corresponds to 0.6 to 1) to generate the fibro-glandular region binary map.

Next, the fibro-glandular region division module 112 may iii) generate a fibro-glandular region division mask based on the fibro-glandular region binary map. For example, the fibro-glandular region division module 112 performs an operation for the breast region division mask 11 and the fibro-glandular region binary map to generate an in-breast fibro-glandular region mask. For example, the fibro-glandular region division module 112 performs a Hadamard product operation for the breast region division mask 11 and the fibro-glandular region binary map to generate the in-breast fibro-glandular region mask. Further, the fibro-glandular region division module 112 may generate the fibro-glandular region division mask 21 based on the in-breast fibro-glandular region mask. The fibro-glandular region division mask is acquired based on a largest region among regions included in the in-breast fibro-glandular region mask. The fibro-glandular region division module 112 leaves only a largest region in the in-breast fibro-glandular region mask to generate the fibro-glandular region division mask 21.

According to an exemplary embodiment of the present disclosure, the glandular tissue region division module 113 may identify a glandular tissue region based on the identified fibro-glandular tissue region. First, i) the glandular tissue region division module 113 may generate the glandular tissue region binary map based on the fibro-glandular region division mask 21. For example, the glandular tissue region division module 113 may receive the fibro-glandular region division mask 21 as an input, and perform image processing for the received the fibro-glandular region division mask 21, and then generate the glandular tissue region binary map. The glandular tissue region division module 113 may generate the glandular tissue region binary map based on the fibro-glandular region division mask 21 after performing the image processing through a plurality of operations disclosed below.

For example, the glandular tissue region division module 113 may ① extract a glandular tissue region of interest mask from the fibro-glandular region division mask 21 in order to generate the glandular tissue region binary map. For example, the glandular tissue region division module 113 may extract the glandular tissue region of interest mask by applying an erosion filter to the fibro-glandular region division mask 21 generated by the fibro-glandular region division module 112. For reference, the erosion filter may process an image by using a small kernel (filter) called a structuring element, and the small kernel primarily has a rectangular or circular shape, and a pixel of the image may be adjusted while the corresponding kernel is overlapped with the image. Further, the glandular tissue region division module 113 may detect a polygonal type region of interest including the glandular tissue region of interest. Further, the glandular tissue region division module 113 may detect a box type region of interest having a minimum size, which includes all glandular tissue region of interest. Further, the glandular tissue region division module 113 may ③ perform histogram based normalization for the ultrasound image inside the polygonal type region of interest. For example, the glandular tissue region division module 113 may perform the histogram based normalization inside the polygonal type (e.g., rectangular) region of interest for the ultrasound image. The glandular tissue region division module 113 may perform the histogram based normalization inside the polygonal type (e.g., rectangular) region of interest for the ultrasound image, and leaves an original image as it is outside the polygonal type (e.g., rectangular) region of interest to generate a region of interest normalization ultrasound image. Further, the glandular tissue region division module 113 ④ performs an operation for the region of interest normalization ultrasound image and the glandular tissue region of interest mask to generate a glandular tissue region of interest image. Further, the glandular tissue region division module 113 performs the Hadamard product operation for the region of interest normalization ultrasound image and the glandular tissue region of interest mask to generate the glandular tissue region of interest image. Further, the glandular tissue region division module 113 ⑤ classifies the glandular tissue region of interest based on a predetermined threshold to generate the glandular tissue region binary map. As an example, a value which is equal to or more than the predetermined threshold is mapped to the glandular tissue region of interest image as 1, and a value which is equal to or less than the predetermined threshold is mapped to the glandular tissue region of interest image as 0 to generate the glandular tissue region binary map.

Next, the glandular tissue region division module 113 may ii) generate a glandular tissue region division mask 31 based on the glandular tissue region binary map. For example, the glandular tissue region division module 113 may generate the glandular tissue region division mask 31 through an image processing task by using the glandular tissue region of interest. The glandular tissue region division module 113 may remove noise by applying a median filter to the glandular tissue region binary map, and generate the glandular tissue region division mask 31.

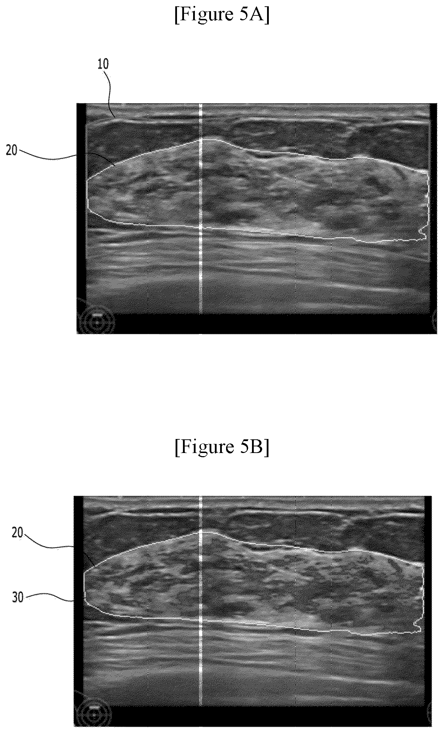

FIG. 5A is a diagram for describing an operation of computing a ratio and a degree of a breast tissue component according to an exemplary embodiment of the present disclosure and FIG. 5B is a diagram for describing an operation of computing a ratio and a degree of a glandular tissue component according to an exemplary embodiment of the present disclosure.

The computing device 100 is configured to quantify at least one of a breast tissue component or a glandular tissue component based on the identified breast region and the identified fibro-glandular tissue region. In the breast ultrasound image, the breast tissue component (BTC) may be divided into three categories (A, B, and C). First, homogenous-fat means a case where most breast tissues are composed of fat. The homogenous-fat breast may correspond to a relatively low grade among occurrence risk grades of a breast cancer. Further, homogenous-fibroglandular means a case where the fat, a fibro tissue, and a fibro-glandular tissue are evenly distributed in the breast tissue. The homogenous-fibroglandular breast may correspond to a median grade among the occurrence risk grades of the breast cancer. Further, heterogeneous means a case where a density and a shade of the breast tissue are uneven. The heterogeneous breast may correspond to a relatively high risk grade among the occurrence risk grades of the breast cancer.

An analysis indicator of another breast tissue a glandular tissue component (GTC), and this may be divided into P1, P2, P3, and P4 grades. Evaluation of the GTC means a ratio of a gray region indicating the glandular tissue to a white region indicating a fibrotic epilepsy in a fibroglandular tissue (FGT) surrounded by subcutaneous and glandular olfactoriae fat in a breast ultrasound. At this time, a fat leaf of the FGT distinguished from the tissue is not included in the GTC evaluation. The glandular tissue composition (GTC) grade may be normally classified into minimum (less than 25% of FGT), mild (25 to 49% of FGT), moderate (50 to 74% of FGT), or marked (75% or more of FGT), and written as abbreviations of P1, P2, P3, and P4 in a reading text. The GTC may reclassify the minimal or mild GTC as "low" and the moderate or marked GTC as "high" based on GTCs constituting 50% of the breast FGT.

The computing device 100 is configured to quantify a breast tissue component based on the identified breast region and the identified fibro-glandular tissue region (S14). For example, referring to FIG. 5A, the computing device 100 may quantify the breast tissue component based on the breast region 10 identified through the breast region division module 111 and the fibro-glandular tissue region 20 identified through the fibro-glandular region division module 112. For example, the computing device 100 may compute a ratio of the breast tissue component by using a pixel value of the fibro-glandular region division mask 21 and a pixel value of the breast region division mask 11. As an example, the computing device 100 may compute the ratio of the breast tissue component based on [a sum of numbers in which the pixel value of the fibro-glandular region division mask 21 is 1 / a sum of numbers in which the pixel value of the breast region division mask 11 is 1]. Further, the computing device 100 may map the ratio of the breast tissue component according to the breast tissue component (BTC) grade. For example, the computing device 100 may map the computed ratio of the breast tissue component to any one of the A, B, and C grades for the breast tissue component (BTC) which are predetermined. As an example, as illustrated in FIG. 5A, the computing device 100 may also express a breast region 10 identified in a line or dotted-line form by using a first color (e.g., a blue), and express the fibro-glandular tissue region 10 identified in the line or dotted-line form by using a second color (e.g., yellow), and provide the breast region 10 and the fibro-glandular tissue region 10 through the user interface.

The computing device 100 is configured to quantify the glandular tissue component by analyzing the identified fibro-glandular tissue region and the glandular tissue region (S15). For example, referring to FIG. 5B, the computing device 100 may quantify the glandular tissue component based on the fibro-glandular tissue region 20 identified through the fibro-glandular region division module 112 and the glandular tissue region 20 identified through the glandular tissue region division module 113. For example, the computing device 100 may compute the ratio of the glandular tissue component based on [a sum of numbers in which the pixel value of the glandular tissue region division mask 31 is 1 / a sum of numbers in which the pixel value of the fibro-glandular region division mask 21 is 1]. Further, the computing device 100 may map the ratio of the glandular tissue component according to the glandular tissue component (GTC) grade. For example, the computing device 100 may map the computed ratio of the glandular tissue component to any one of the P1, P2, P3, and P4 grades for the glandular tissue component (GTC) which are predetermined. As an example, as illustrated in FIG. 5B, the computing device 100 may also express the fibro-glandular tissue region 10 identified in the line or dotted-line form by using the second color (e.g., yellow), and express the glandular tissue region 30 identified in the line or dotted-line form by using a third color (e.g., red), and provide the fibro-glandular tissue region 10 and the glandular tissue region 30 through a user interface.

According to an exemplary embodiment of the present disclosure, the computing device 100 may select a representative image from ultrasound images (S16). As an example, the computing device 100 may extract a significant image from breast ultrasound images including noise. The computing device 100 may determine whether the breast ultrasound image is suitable for evaluating the breast component. For example, the computing device 100 may perform evaluation for the breast ultrasound image related to whether the breast ultrasound image is suitable for a calculation level of the breast tissue component (BTC) and the glandular tissue component (GTC). Further, the computing device 100 may select K representative images for evaluating the breast component based on a determination result of determining whether the breast ultrasound image is suitable for evaluating the breast component. For example, the computing device 100 may select the K representative images by determining the clarity of the image, a noise level of the image, a shaking degree of the image, a range (e.g., checking whether an image of a sufficient range including the entire breast is included) of the image, an angle of the image, a quality of the image, a distribution of the breast tissue, a density and a shade of the breast tissue, etc., with respect to the acquired breast ultrasound images. However, a determination condition for selecting the representative image is not limited thereto. Further, the computing device 100 may calculate the ratios and the grades for the breast tissue component (BTC) and the glandular tissue component (GTC) by the unit of Exam by averaging K representative images which are selected. For reference, when examining a patient by using a medical imaging device (e.g., ultrasound, X-ray, MRI, etc.), grouping images generated in the corresponding device into one "Exam" may be referred to as the Exam unit.

Hereinafter, an operation flow of the present disclosure will be described in brief based on the contents described in detail.

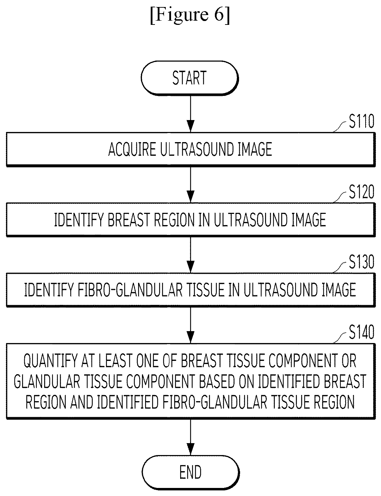

FIG. 6 is a flowchart illustrating a method for quantifying a breast component according to an exemplary embodiment of the present disclosure.

The method for quantifying the breast composition illustrated in FIG. 6 may be performed by the computing device 100 described above. Hereinafter, in spite of contents omitted below, the contents described regarding the computing device 100 may also be similarly applied to a description of the method for quantifying the breast composition.

Referring to FIG. 6, the method for quantifying the breast composition includes a step (S110) of acquiring an ultrasound image, a step (S120) of identifying a breast region in the ultrasound image, a step (S130) of identifying a fibro-glandular tissue region in the ultrasound image, and a step (S140) of quantifying at least one of a breast tissue component or a glandular tissue component based on the identified breast region and the identified fibro-glandular tissue region.

Step S110 is a step of acquiring the ultrasound image. As an example, the computing device 100 may scan a breast by using ultrasound equipment (e.g., an ultrasound probe, etc.), and acquire the ultrasound image.

Step S120 is a step of identifying the breast region in the ultrasound image. Step S120 above may include a step of generating a breast inference region mask in the ultrasound image by using a deep learning model, a step of generating a breast region binary map based on the generated breast inference region mask, and generating a breast region division mask based on the breast region binary map. Here, the breast region division mask may be acquired based on a largest region among regions included in the breast region binary map.

Step S130 is a step of identifying a fibro-glandular tissue region in the ultrasound image. Step S130 above may include a step of generating a fibro-glandular inference region mask in the ultrasound image by using the deep learning model, a step of generating a fibro-glandular region binary map based on the generated fibro-glandular inference region mask, and a step of generating a fibro-glandular region division mask based on the fibro-glandular region binary map.

Step S140 is a step of quantifying at least one of a breast tissue component or a glandular tissue component based on the identified breast region and the identified fibro-glandular tissue region. Step S140 may include a step of identifying a glandular tissue region based on the identified fibro-glandular tissue region, and a step of quantifying the glandular tissue component by analyzing the identified fibro-glandular tissue region and the identified glandular tissue region. Further, step S140 may include a step of computing a ratio of the breast tissue component by using a pixel value of the fibro-glandular region division mask and a pixel value of the breast region division mask, and a step of mapping the ratio of the breast tissue component according to a breast tissue component (BTC) grade. Further, step S140 may include a step of computing the ratio of the glandular tissue component by using a pixel value of the glandular tissue region division mask and the pixel value of the fibro-glandular region division mask, and a step of mapping the ratio of the glandular tissue component according to a glandular tissue component (GTC) grade.

The steps mentioned in the above description may be further divided into additional steps or combined into fewer steps, depending on the implementation of the present disclosure. In addition, some steps may be omitted as needed, and the order between the steps may be changed.

Disclosed is a computer readable medium storing the data structure according to the present invention.

The data structure may refer to the organization, management, and storage of data that enables efficient access to and modification of data. The data structure may refer to the organization of data for solving a specific problem (e.g., data search, data storage, data modification in the shortest time). The data structures may be defined as physical or logical relationships between data elements, designed to support specific data processing functions. The logical relationship between data elements may include a connection between data elements that the user defines. The physical relationship between data elements may include an actual relationship between data elements physically stored on a computer-readable storage medium (e.g., persistent storage device). The data structure may specifically include a set of data, a relationship between the data, a function which may be applied to the data, or instructions. Through an availablely designed data structure, a computing device can perform operations while using the resources of the computing device to a minimum. Specifically, the computing device can increase the efficiency of operation, read, insert, delete, compare, exchange, and search through the availablely designed data structure.

The data structure may be divided into a linear data structure and a non-linear data structure according to the type of data structure. The linear data structure may be a structure in which only one data is connected after one data. The linear data structure may include a list, a stack, a queue, and a deque. The list may mean a series of data sets in which an order exists internally. The list may include a linked list. The linked list may be a data structure in which data is connected in a scheme in which each data is linked in a row with a pointer. In the linked list, the pointer may include link information with next or previous data. The linked list may be represented as a single linked list, a double linked list, or a circular linked list depending on the type. The stack may be a data listing structure with limited access to data. The stack may be a linear data structure that may process (e.g., insert or delete) data at only one end of the data structure. The data stored in the stack may be a data structure (LIFO-Last in First Out) in which the data is input last and output first. The queue is a data listing structure that may access data limitedly and unlike a stack, the queue may be a data structure (FIFO-First in First Out) in which late stored data is output late. The deque may be a data structure capable of processing data at both ends of the data structure.

The non-linear data structure may be a structure in which a plurality of data are connected after one data. The non-linear data structure may include a graph data structure. The graph data structure may be defined as a vertex and an edge, and the edge may include a line connecting two different vertices. The graph data structure may include a tree data structure. The tree data structure may be a data structure in which there is one path connecting two different vertices among a plurality of vertices included in the tree. That is, the tree data structure may be a data structure that does not form a loop in the graph data structure.

In the present disclosure, a network function, an artificial neural network, and a neural network may be used to be exchangeable. From here on, it will be described uniformly using neural networks.

The data structure may include the neural network. In addition, the data structures, including the neural network, may be stored in a computer readable medium. The data structure including the neural network may also include data preprocessed for processing by the neural network, data input to the neural network, weights of the neural network, hyper parameters of the neural network, data obtained from the neural network, an active function associated with each node or layer of the neural network, and a loss function for training the neural network. The data structure including the neural network may include predetermined components of the components disclosed above. In other words, the data structure including the neural network may include all of data preprocessed for processing by the neural network, data input to the neural network, weights of the neural network, hyper parameters of the neural network, data obtained from the neural network, an active function associated with each node or layer of the neural network, and a loss function for training the neural network or a combination thereof. In addition to the above-described configurations, the data structure including the neural network may include predetermined other information that determines the characteristics of the neural network. In addition, the data structure may include all types of data used or generated in the calculation process of the neural network, and is not limited to the above. The computer readable medium may include a computer readable recording medium and/or a computer readable transmission medium. The neural network may be generally constituted by an aggregate of calculation units which are mutually connected to each other, which may be called nodes. The nodes may also be called neurons. The neural network is configured to include one or more nodes.

The data structure may include data input into the neural network. The data structure including the data input into the neural network may be stored in the computer readable medium. The data input to the neural network may include training data input in a neural network training process and/or input data input to a neural network in which training is completed. The data input to the neural network may include preprocessed data and/or data to be preprocessed. The preprocessing may include a data processing process for inputting data into the neural network. Therefore, the data structure may include data to be preprocessed and data generated by preprocessing. The data structure is just an example and the present disclosure is not limited thereto.

The data structure may include the weight of the neural network (in the present disclosure, the weight and the parameter may be used as the same meaning). In addition, the data structures, including the weight of the neural network, may be stored in the computer readable medium. The neural network may include a plurality of weights. The weight may be variable and the weight is variable by a user or an algorithm in order for the neural network to perform a desired function. For example, when one or more input nodes are mutually connected to one output node by the respective links, the output node may determine a data value output from an output node based on values input in the input nodes connected with the output node and the weights set in the links corresponding to the respective input nodes. The data structure is just an example and the present disclosure is not limited thereto.

As a non-limiting example, the weight may include a weight which varies in the neural network training process and/or a weight in which neural network training is completed. The weight which varies in the neural network training process may include a weight at a time when a training cycle starts and/or a weight that varies during the training cycle. The weight in which the neural network training is completed may include a weight in which the training cycle is completed. Accordingly, the data structure including the weight of the neural network may include a data structure including the weight which varies in the neural network training process and/or the weight in which neural network training is completed. Accordingly, the above-described weight and/or a combination of each weight are included in a data structure including a weight of a neural network. The data structure is just an example and the present disclosure is not limited thereto.

The data structure including the weight of the neural network may be stored in the computer-readable storage medium (e.g., memory, hard disk) after a serialization process. Serialization may be a process of storing data structures on the same or different computing devices and later reconfiguring the data structure and converting the data structure to a form that may be used. The computing device may serialize the data structure to send and receive data over the network. The data structure including the weight of the serialized neural network may be reconfigured in the same computing device or another computing device through deserialization. The data structure including the weight of the neural network is not limited to the serialization. Furthermore, the data structure including the weight of the neural network may include a data structure (for example, B-Tree, Trie, m-way search tree, AVL tree, and Red-Black Tree in a nonlinear data structure) to increase the efficiency of operation while using resources of the computing device to a minimum. The above-described matter is just an example and the present disclosure is not limited thereto.

The data structure may include hyper-parameters of the neural network. In addition, the data structures, including the hyper-parameters of the neural network, may be stored in the computer readable medium. The hyper-parameter may be a variable which may be varied by the user. The hyper-parameter may include, for example, a learning rate, a cost function, the number of training cycle iterations, weight initialization (for example, setting a range of weight values to be subjected to weight initialization), and Hidden Unit number (e.g., the number of hidden layers and the number of nodes in the hidden layer). The data structure is just an example and the present disclosure is not limited thereto.

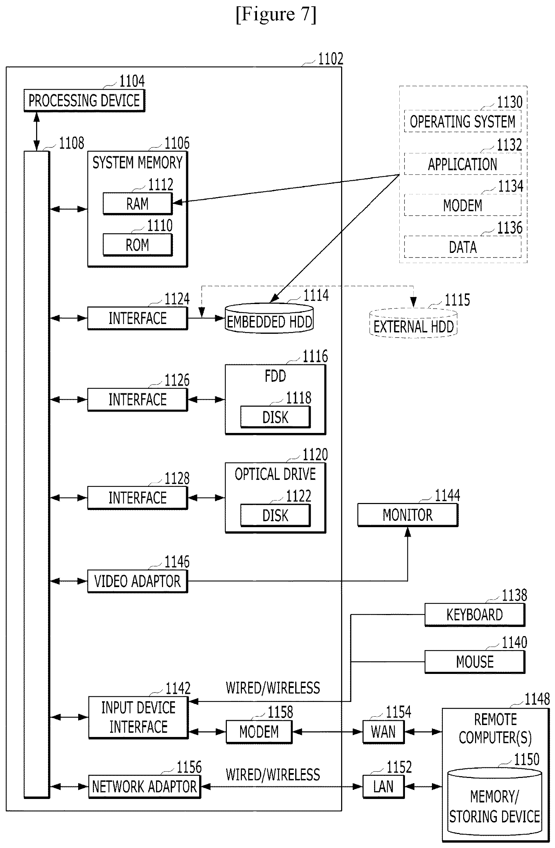

FIG. 7 is a normal and schematic view of an exemplary computing environment in which the exemplary embodiments of the present disclosure may be implemented.

It is described above that the present disclosure may be generally implemented by the computing device, but those skilled in the art will well know that the present disclosure may be implemented in association with a computer executable command which may be executed on one or more computers and/or in combination with other program modules and/or a combination of hardware and software.

In general, the program module includes a routine, a program, a component, a data structure, and the like that execute a specific task or implement a specific abstract data type. Further, it will be well appreciated by those skilled in the art that the method of the present disclosure can be implemented by other computer system configurations including a personal computer, a handheld computing device, microprocessor-based or programmable home appliances, and others (the respective devices may operate in connection with one or more associated devicesas well as a single-processor or multi-processor computer system, a mini computer, and a main frame computer.

The exemplary embodiments described in the present disclosure may also be implemented in a distributed computing environment in which predetermined tasks are performed by remote processing devices connected through a communication network. In the distributed computing environment, the program module may be positioned in both local and remote memory storage devices.

The computer generally includes various computer readable media. Media accessible by the computer may be computer readable media regardless of types thereof and the computer readable media include volatile and non-volatile media, transitory and non-transitory media, and mobile and non-mobile media. As a non-limiting example, the computer readable media may include both computer readable storage media and computer readable transmission media. The computer readable storage media include volatile and non-volatile media, transitory and non-transitory media, and mobile and non-mobile media implemented by a predetermined method or technology for storing information such as a computer readable instruction, a data structure, a program module, or other data. The computer readable storage media include a RAM, a ROM, an EEPROM, a flash memory or other memory technologies, a CD-ROM, a digital video disk (DVD) or other optical disk storage devices, a magnetic cassette, a magnetic tape, a magnetic disk storage device or other magnetic storage devices or predetermined other media which may be accessed by the computer or may be used to store desired information, but are not limited thereto.

The computer readable transmission media generally implement the computer readable command, the data structure, the program module, or other data in a carrier wave or a modulated data signal such as other transport mechanism and include all information transfer media. The term "modulated data signal" means a signal acquired by setting or changing at least one of characteristics of the signal so as to encode information in the signal. As a non-limiting example, the computer readable transmission media include wired media such as a wired network or a direct-wired connection and wireless media such as acoustic, RF, infrared and other wireless media. A combination of anymedia among the aforementioned media is also included in a range of the computer readable transmission media.

An exemplary environment 1100 that implements various aspects of the present disclosure including a computer 1102 is shown and the computer 1102 includes a processing device 1104, a system memory 1106, and a system bus 1108. The system bus 1108 connects system components including the system memory 1106 (not limited thereto) to the processing device 1104. The processing device 1104 may be a predetermined processor among various commercial processors. A dual processor and other multi-processor architectures may also be used as the processing device 1104.

The system bus 1108 may be any one of several types of bus structures which may be additionally interconnected to a local bus using any one of a memory bus, a peripheral device bus, and various commercial bus architectures. The system memory 1106 includes a read only memory (ROM) 1110 and a random access memory (RAM) 1112. A basic input/output system (BIOS) is stored in the non-volatile memories 1110 including the ROM, the EPROM, the EEPROM, and the like and the BIOS includes a basic routine that assists in transmitting information among components in the computer 1102 at a time such as in-starting. The RAM 1112 may also include a high-speed RAM including a static RAM for caching data, and the like.

The computer 1102 also includes an interior hard disk drive (HDD) 1114 (for example, EIDE and SATA), in which the interior hard disk drive 1114 may also be configured for an exterior purpose in an appropriate chassis (not illustrated), a magnetic floppy disk drive (FDD) 1116 (for example, for reading from or writing in a mobile diskette 1118), and an optical disk drive 1120 (for example, for reading a CD-ROM disk 1122 or reading from or writing in other high-capacity optical media such as the DVD, and the like). The hard disk drive 1114, the magnetic disk drive 1116, and the optical disk drive 1120 may be connected to the system bus 1108 by a hard disk drive interface 1124, a magnetic disk drive interface 1126, and an optical drive interface 1128, respectively. An interface 1124 for implementing an exterior drive includes at least one of a universal serial bus (USB) and an IEEE 1394 interface technology or both of them.

The drives and the computer readable media associated therewith provide non-volatile storage of the data, the data structure, the computer executable instruction, and others. In the case of the computer 1102, the drives and the media correspond to storing of predetermined data in an appropriate digital format. In the description of the computer readable media, the mobile optical media such as the HDD, the mobile magnetic disk, and the CD or the DVD are mentioned, but it will be well appreciated by those skilled in the art that other types of media readable by the computer such as a zip drive, a magnetic cassette, a flash memory card, a cartridge, and others may also be used in an exemplary operating environment and further, the predetermined media may include computer executable commands for executing the methods of the present disclosure. Multiple program modules including an operating system 1130, one or more application programs 1132, other program module 1134, and program data 1136 may be stored in the drive and the RAM 1112. All or some of the operating system, the application, the module, and/or the data may also be cached in the RAM 1112. It will be well appreciated that the present disclosure may be implemented in operating systems which are commercially usable or a combination of the operating systems.

A user may input instructions and information in the computer 1102 through one or more wired/wireless input devices, for example, pointing devices such as a keyboard 1138 and a mouse 1140. Other input devices (not illustrated) may include a microphone, an IR remote controller, a joystick, a game pad, a stylus pen, a touch screen, and others. These and other input devices are often connected to the processing device 1104 through an input device interface 1142 connected to the system bus 1108, but may be connected by other interfaces including a parallel port, an IEEE 1394 serial port, a game port, a USB port, an IR interface, and others.

A monitor 1144 or other types of display devices are also connected to the system bus 1108 through interfaces such as a video adapter 1146, and the like. In addition to the monitor 1144, the computer generally includes other peripheral output devices (not illustrated) such as a speaker, a printer, others.

The computer 1102 may operate in a networked environment by using a logical connection to one or more remote computers including remote computer(s) 1148 through wired and/or wireless communication. The remote computer(s) 1148 may be a workstation, a computing device computer, a router, a personal computer, a portable computer, a micro-processor based entertainment apparatus, a peer device, or other general network nodes and generally includes multiple components or all of the components described with respect to the computer 1102, but only a memory storage device 1150 is illustrated for brief description. The illustrated logical connection includes a wired/wireless connection to a local area network (LAN) 1152 and/or a larger network, for example, a wide area network (WAN) 1154. The LAN and WAN networking environments are general environments in offices and companies and facilitate an enterprise-wide computer network such as Intranet, and all of them may be connected to a worldwide computer network, for example, the Internet.

When the computer 1102 is used in the LAN networking environment, the computer 1102 is connected to a local network 1152 through a wired and/or wireless communication network interface or an adapter 1156. The adapter 1156 may facilitate the wired or wireless communication to the LAN 1152 and the LAN 1152 also includes a wireless access point installed therein in order to communicate with the wireless adapter 1156. When the computer 1102 is used in the WAN networking environment, the computer 1102 may include a modem 1158 or has other means that configure communication through the WAN 1154 such as connection to a communication computing device on the WAN 1154 or connection through the Internet. The modem 1158 which may be an internal or external and wired or wireless device is connected to the system bus 1108 through the serial port interface 1142. In the networked environment, the program modules described with respect to the computer 1102 or some thereof may be stored in the remote memory/storage device 1150. It will be well known that an illustrated network connection is exemplary and other means configuring a communication link among computers may be used.

The computer 1102 performs an operation of communicating with predetermined wireless devices or entities which are disposed and operated by the wireless communication, for example, the printer, a scanner, a desktop and/or a portable computer, a portable data assistant (PDA), a communication satellite, predetermined equipment or place associated with a wireless detectable tag, and a telephone. This at least includes wireless fidelity (Wi-Fi) and Bluetooth wireless technology. Accordingly, communication may be a predefined structure like the network in the related art or just ad hoc communication between at least two devices.

The wireless fidelity (Wi-Fi) enables connection to the Internet, and the like without a wired cable. The Wi-Fi is a wireless technology such as the device, for example, a cellular phone which enables the computer to transmit and receive data indoors or outdoors, that is, anywhere in a communication range of a base station. The Wi-Fi network uses a wireless technology called IEEE 802.11(a, b, g, and others) in order to provide safe, reliable, and high-speed wireless connection. The Wi-Fi may be used to connect the computers to each other or the Internet and the wired network (using IEEE 802.3 or Ethernet). The Wi-Fi network may operate, for example,at a data rate of 11 Mbps (802.11a) or 54 Mbps (802.11b) in unlicensed 2.4 and 5GHz wireless bands or operate in a product including both bands (dual bands).

It will be appreciated by those skilled in the art that information and signals may be expressed by using various different predetermined technologies and techniques. For example, data, instructions, commands, information, signals, bits, symbols, and chips which may be referred in the above description may be expressed by voltages, currents, electromagnetic waves, magnetic fields or particles, optical fields or particles, or predetermined combinations thereof.

It may be appreciated by those skilled in the art that various exemplary logical blocks, modules, processors, means, circuits, and algorithm steps described in association with the exemplary embodiments disclosed herein may be implemented by electronic hardware, various types of programs or design codes (for easy description, herein, designated as software), or a combination of all of them. In order to clearly describe the intercompatibility of the hardware and the software, various exemplary components, blocks, modules, circuits, and steps have been generally described above in association with functions thereof. Whether the functions are implemented as the hardware or software depends on design restrictions given to a specific application and an entire system. Those skilled in the art of the present disclosure may implement functions described by various methods with respect to each specific application, but it should not be interpreted that the implementation determination departs from the scope of the present disclosure.

Various exemplary embodiments presented herein may be implemented as manufactured articles using a method, a device, or a standard programming and/or engineering technique. The term manufactured article includes a computer program, a carrier, or a medium which is accessible by a predetermined computer-readable storage device. For example, a computer-readable storage medium includes a magnetic storage device (for example, a hard disk, a floppy disk, a magnetic strip, or the like), an optical disk (for example, a CD, a DVD, or the like), a smart card, and a flash memory device (for example, an EEPROM, a card, a stick, a key drive, or the like), but is not limited thereto. Further, various storage media presented herein include one or more devices and/or other machine-readable media for storing information.

It will be appreciated that a specific order or a hierarchical structure of steps in the presented processes is one example of exemplary accesses. It will be appreciated that the specific order or the hierarchical structure of the steps in the processes within the scope of the present disclosure may be rearranged based on design priorities. Appended method claims provide elements of various steps in a sample order, but the method claims are not limited to the presented specific order or hierarchical structure.

The description of the presented exemplary embodiments is provided so that those skilled in the art of the present disclosure use or implement the present disclosure. Various modifications of the exemplary embodiments will be apparent to those skilled in the art and general principles defined herein can be applied to other exemplary embodiments without departing from the scope of the present disclosure. Therefore, the present disclosure is not limited to the exemplary embodiments presented herein, but should be interpreted within the widest range which is coherent with the principles and new features presented herein.

## Claims

1. A method for quantifying a breast composition performed by a computing device, the method comprising:
acquiring an ultrasound image (S10, S110);
identifying a breast region in the ultrasound image (S11, S120);
identifying a fibro-glandular tissue region in the ultrasound image, comprising generating at least one of a fibro-glandular inference region mask and a fibro-glandular region binary map using a deep learning model (S12, S130); and
quantifying at least one of a breast tissue component (S14) or a glandular tissue component (S15) based on the identified breast region and the identified fibro-glandular tissue region, comprising performing histogram-based normalization within an in-breast region of interest to determine a glandular tissue component ratio (S140),
**characterized in that** the identifying of the breast region includes:
generating a breast inference region mask in the ultrasound image using a deep learning model,
generating a breast region binary map based on the generated breast inference region mask, and
generating a breast region division mask (11) based on a largest region among regions included in the breast region binary map,
wherein the identifying of the fibro-glandular tissue region in the ultrasound image includes:
generating a fibro-glandular inference region mask in the ultrasound image using a deep learning model;
generating a fibro-glandular region binary map based on the generated fibro-glandular inference region mask; and
generating a fibro-glandular region division mask (21) based on the fibro-glandular region binary map.

2. The method of claim 1, wherein the breast inference region mask includes a breast region probability map, and
wherein the generating of the breast region binary map based on the generated breast inference region mask includes:
generating the breast region binary map by classifying the breast region probability map based on a predetermined threshold.

3. The method of claim 1, wherein the fibro-glandular inference region mask includes a fibro-glandular region probability map, and
wherein the generating of the fibro-glandular region binary map based on the generated fibro-glandular inference region mask includes:
generating the fibro-glandular region binary map by classifying the fibro-glandular region probability map based on a predetermined threshold.

4. The method of claim 1, wherein the generating of the fibro-glandular region division mask (21) based on the fibro-glandular region binary map includes:
generating an in-breast fibro-glandular region mask by performing an operation for the breast region division mask (11) and the fibro-glandular region binary map; and
generating the fibro-glandular region division mask (21) based on the in-breast fibro-glandular region mask.

5. The method of claim 4, wherein the fibro-glandular region division mask is acquired based on a largest region among regions included in the in-breast fibro-glandular region mask.

6. The method of claim 1, wherein the quantifying of at least one of the breast tissue component (S14) or the glandular tissue component (S15) based on the identified breast region and the identified fibro-glandular tissue region (S140) includes:
identifying a glandular tissue region based on the identified fibro-glandular tissue region; and
quantifying the glandular tissue component by analyzing the identified fibro-glandular tissue region and the identified glandular tissue region.

7. The method of claim 6, wherein the identifying of the glandular tissue region based on the identified fibro-glandular tissue region includes:
generating a glandular tissue region binary map based on a fibro-glandular region division mask, and
generating a glandular tissue region division mask (31) based on the glandular tissue region binary map.

8. The method of claim 7, wherein the generating of the glandular tissue region binary map based on the fibro-glandular region division mask includes:
extracting a glandular tissue region of interest mask from the fibro-glandular region division mask;
detecting a polygonal type region of interest including the glandular tissue region of interest;
performing histogram based normalization for an ultrasound image inside the polygonal type region of interest;
generating a region of interest normalization ultrasound image by using the ultrasound image inside the polygonal type region of interest in which the histogram based normalization is performed and an ultrasound image outside the polygonal type region of interest;
generating a glandular tissue region of interest image by performing an operation for the region of interest normalization ultrasound image and the glandular tissue region of interest mask; and
generating the glandular tissue region binary map by classifying the glandular tissue region of interest image based on a predetermined threshold.

9. The method of claim 1, wherein the quantifying of at least one of the breast tissue component (S14) or the glandular tissue component (S15) based on the identified breast region and the identified fibro-glandular tissue region (S140) includes:
computing a ratio of the breast tissue component by using a pixel value of a fibro-glandular region division mask and a pixel value of a breast region division mask, and
mapping the ratio of the breast tissue component according to a breast tissue component (BTC) grade.

10. The method of claim 1, wherein the quantifying of at least one of the breast tissue component (S14) or the glandular tissue component (S15) based on the identified breast region and the identified fibro-glandular tissue region (S140) includes:
computing a ratio of the glandular tissue component by using a pixel value of a glandular tissue region division mask (31) and a pixel value of a fibro-glandular region division mask (21), and
mapping the ratio of the glandular tissue component according to a glandular tissue component (GTC) grade.

11. A non-transitory computer-readable storage medium comprising a computer program, wherein when the computer program is executed by one or more processors, the computer program allows the one or more processors to perform following operations for quantifying a breast composition, the operations comprising:
an operation of acquiring an ultrasound image (S10, S110);
an operation of identifying a breast region in the ultrasound image (S11, S120);
an operation of identifying a fibro-glandular tissue region in the ultrasound image, comprising generating at least one of a fibro-glandular inference region mask and a fibro-glandular region binary map using a deep learning model (S12, S130); and
an operation of quantifying at least one of a breast tissue component (S14) or a glandular tissue component (S15) based on the identified breast region and the identified fibro-glandular tissue (S140) region, comprising performing histogram-based normalization within an in-breast region of interest to determine a glandular tissue component ratio(S140),
**characterized in that** the operation of identifying of the breast region includes:
an operation of generating a breast inference region mask in the ultrasound image using a deep learning model,
an operation of generating a breast region binary map based on the generated breast inference region mask, and
an operation of generating a breast region division mask based on a largest region among regions included in the breast region binary map,
wherein the operation of identifying of the fibro-glandular tissue region in the ultrasound image includes:
an operation of generating a fibro-glandular inference region mask in the ultrasound image using a deep learning model;
an operation of generating a fibro-glandular region binary map based on the generated fibro-glandular inference region mask; and
an operation of generating a fibro-glandular region division mask (21) based on the fibro-glandular region binary map.

12. A computing device comprising:
at least one processor; and
a memory,
wherein the at least one processor is configured to:
acquire an ultrasound image (S10, S110),
identify a breast region in the ultrasound image (S11, S120),
identify a fibro-glandular tissue region in the ultrasound image, comprising generating at least one of a fibro-glandular inference region mask and a fibro-glandular region binary map using a deep learning model (S12, S130); and
quantify at least one of a breast tissue component (S14) or a glandular tissue component (S15) based on the identified breast region and the identified fibro-glandular tissue region, comprising performing histogram-based normalization within an in-breast region of interest to determine a glandular tissue component ratio(S140),
**characterized in that** the identifying of the breast region includes:
generating a breast inference region mask in the ultrasound image using a deep learning model,
generating a breast region binary map based on the generated breast inference region mask, and
generating a breast region division mask based on a largest region among regions included in the breast region binary map,
wherein the identifying of the fibro-glandular tissue region in the ultrasound image includes:
generating a fibro-glandular inference region mask in the ultrasound image using a deep learning model;
generating a fibro-glandular region binary map based on the generated fibro-glandular inference region mask; and
generating a fibro-glandular region division mask (21) based on the fibro-glandular region binary map.

## Patentansprüche

1. Verfahren zur Quantifizierung einer Brustzusammensetzung, das von einer Rechenvorrichtung durchgeführt wird, wobei das Verfahren umfasst:
Erfassen eines Ultraschallbildes (S10, S110);
Erkennen einer Brustregion im Ultraschallbild (S11, S120);
Erkennen einer fibro-glandulären Geweberegion im Ultraschallbild, umfassend Erzeugen zumindest einer von einer fibro-glandulären Inferenzregionsmaske und einer fibro-glandulären Regionsbinärkarte unter Verwendung eines Deep-Learning-Modelles (S12, S130); und
Quantifizieren zumindest eines von einem Brustgewebeanteil (S14) oder einem Drüsengewebeanteil (S15) aufgrund der erkannten Brustregion und der erkannten fibro-glandulären Geweberegion, umfassend Durchführen von Histogramm-basierter Normalisierung innerhalb einer brustinternen Interessenregion, um ein Drüsengewebeanteilverhältnis (S140) zu bestimmen,
**dadurch gekennzeichnet, dass** das Erkennen der Brustregion umfasst:
Erzeugen einer Brustinferenzregionsmaske im Ultraschallbild unter Verwendung eines Deep-Learning-Modelles,
Erzeugen einer Brustregionsbinärkarte aufgrund der erzeugten Brustinferenzregionsmaske, und
Erzeugen einer Brustregionsteilungsmaske (11) aufgrund einer größten Region unter den Regionen, die in der Brustregionsbinärkarte umfasst sind,
worin das Erkennen der fibro-glandulären Geweberegion im Ultraschallbild umfasst:
Erzeugen einer fibro-glandulären Inferenzregionsmaske im Ultraschallbild unter Verwendung eines Deep-Learning-Modelles;
Erzeugen einer fibro-glandulären Regionsbinärkarte aufgrund der erzeugten fibro-glandulären Inferenzregionsmaske; und
Erzeugen einer fibro-glandulären Regionsteilungsmaske (21) aufgrund der fibro-glandulären Regionsbinärkarte.

2. Verfahren nach Anspruch 1, worin die Brustinferenzregionsmaske eine Brustregionswahrscheinlichkeitskarte umfasst, und
worin das Erzeugen der Brustregionsbinärkarte aufgrund der erzeugten Brustinferenzregionsmaske umfasst:
Erzeugen der Brustregionsbinärkarte durch Klassifizieren der Brustregionswahrscheinlichkeitskarte aufgrund einer vorbestimmten Schwelle.

3. Verfahren nach Anspruch 1, worin die fibro-glanduläre Inferenzregionsmaske eine fibro-glanduläre Regionswahrscheinlichkeitskarte umfasst, und
worin das Erzeugen der fibro-glandulären Regionsbinärkarte aufgrund der erzeugten fibro-glandulären Inferenzregionsmaske umfasst:
Erzeugen der fibro-glandulären Regionsbinärkarte durch Klassifizieren der fibro-glandulären Regionswahrscheinlichkeitskarte aufgrund einer vorbestimmten Schwelle.

4. Verfahren nach Anspruch 1, worin das Erzeugen der fibro-glandulären Regionsteilungsmaske (21) aufgrund der fibro-glandulären Regionsbinärkarte umfasst:
Erzeugen einer brustinternen fibro-glandulären Regionsmaske durch Durchführen eines Vorgangs für die Brustregionsteilungsmaske (11) und die fibro-glanduläre Regionsbinärkarte; und
Erzeugen der fibro-glandulären Regionsteilungsmaske (21) aufgrund der brustinternen fibro-glandulären Regionsmaske.

5. Verfahren nach Anspruch 4, worin die fibro-glanduläre Regionsteilungsmaske aufgrund einer größten Region unter Regionen, die in der brustinternen fibro-glandulären Regionsmaske umfasst sind, erfasst wird.

6. Verfahren nach Anspruch 1, worin das Quantifizieren zumindest eines von dem Brustgewebeanteil (S14) oder dem Drüsengewebeanteil (S15) aufgrund der erkannten Brustregion und der erkannten fibro-glandulären Geweberegion (S140) umfasst:
Erkennen einer Drüsengeweberegion aufgrund der erkannten fibro-glandulären Geweberegion; und
Quantifizieren des Drüsengewebeanteils durch Analysieren der erkannten fibro-glandulären Geweberegion und der erkannten Drüsengeweberegion.

7. Verfahren nach Anspruch 6, worin das Erkennen der Drüsengeweberegion aufgrund der erkannten fibro-glandulären Geweberegion umfasst:
Erzeugen einer Drüsengeweberegionsbinärkarte aufgrund einer fibro-glandulären Regionsteilungsmaske, und
Erzeugen einer Drüsengeweberegionsteilungsmaske (31) aufgrund der Drüsengeweberegionsbinärkarte.

8. Verfahren nach Anspruch 7, worin das Erzeugen der Drüsengeweberegionsbinärkarte aufgrund der fibro-glandulären Regionsteilungsmaske umfasst:
Extrahieren einer Drüsengewebeinteressenregionsmaske aus der fibro-glandulären Regionsteilungsmaske;
Detektieren einer Interessenregion des polygonalen Typs, die die Drüsengewebeinteressenregion umfasst;
Durchführen von Histogramm-basierter Normalisierung für ein Ultraschallbild innerhalb der Interessenregion des polygonalen Typs;
Erzeugen eines Interessenregionsnormalisierungsultraschallbildes durch Verwendung des Ultraschallbildes innerhalb der Interessenregion des polygonalen Typs, in der die Histogramm-basierte Normalisierung durchgeführt wird, und eines Ultraschallbildes außerhalb der Interessenregion des polygonalen Typs;
Erzeugen eines Drüsengewebeinteressenregionsbildes durch Durchführen eines Vorgangs für das Interessenregionsnormalisierungsultraschallbild und die Drüsengewebeinteressenregionsmaske; und
Erzeugen der Drüsengeweberegionsbinärkarte durch Klassifizieren des Drüsengewebeinteressenregionsbildes aufgrund einer vorbestimmten Schwelle.

9. Verfahren nach Anspruch 1, worin das Quantifizieren zumindest eines von dem Brustgewebeanteil (S14) oder dem Drüsengewebeanteil (S15) aufgrund der erkannten Brustregion und der erkannten fibro-glandulären Geweberegion (S140) umfasst:
Berechnen eines Verhältnisses des Brustgewebeanteils durch Verwendung eines Pixelwertes einer fibro-glandulären Regionsteilungsmaske und eines Pixelwertes einer Brustregionsteilungsmaske, und
Zuordnen des Verhältnisses des Brustgewebeanteils gemäß einem Brustgewebeanteils(BTC)-Grad.

10. Verfahren nach Anspruch 1, worin das Quantifizieren zumindest eines von dem Brustgewebeanteil (S14) oder dem Drüsengewebeanteil (S15) aufgrund der erkannten Brustregion und der erkannten fibro-glandulären Geweberegion (S140) umfasst:
Berechnen eines Verhältnisses des Drüsengewebeanteils durch Verwendung eines Pixelwertes einer Drüsengeweberegionsteilungsmaske (31) und eines Pixelwertes einer fibro-glandulären Regionsteilungsmaske (21), und
Zuordnen des Verhältnisses des Drüsengewebeanteils gemäß einem Drüsengewebeanteils(GTC)-Grad.

11. Nicht-flüchtiges computerlesbares Speichermedium umfassend ein Computerprogramm, worin, wenn das Computerprogramm von einem oder mehreren Prozessoren ausgeführt wird, das Computerprogramm dem einen oder den mehreren Prozessoren ermöglicht, die folgenden Vorgänge zum Quantifizieren einer Brustzusammensetzung durchzuführen, wobei die Vorgänge umfassen:
einen Vorgang des Erfassens eines Ultraschallbildes (S10, S110);
einen Vorgang des Erkennens einer Brustregion im Ultraschallbild (S11, S120);
einen Vorgang des Erkennens einer fibro-glandulären Geweberegion im Ultraschallbild, umfassend Erzeugen zumindest einer von einer fibro-glandulären Inferenzregionsmaske und einer fibro-glandulären Regionsbinärkarte unter Verwendung eines Deep-Learning-Modelles (S12, S130); und
einen Vorgang des Quantifizierens zumindest eines von einem Brustgewebeanteil (S14) oder einem Drüsengewebeanteil (S15) aufgrund der erkannten Brustregion und der erkannten fibro-glandulären Geweberegion (S140), umfassend Durchführen von Histogramm-basierter Normalisierung innerhalb einer brustinternen Interessenregion, um ein Drüsengewebeanteilverhältnis zu bestimmen (S140),
**dadurch gekennzeichnet, dass** der Vorgang des Erkennens der Brustregion umfasst:
einen Vorgang des Erzeugens einer Brustinferenzregionsmaske im Ultraschallbild unter Verwendung eines Deep-Learning-Modelles,
einen Vorgang des Erzeugens einer Brustregionsbinärkarte aufgrund der erzeugten Brustinferenzregionsmaske, und
einen Vorgang des Erzeugens einer Brustregionsteilungsmaske aufgrund einer größten Region unter den Regionen, die in der Brustregionsbinärkarte umfasst sind,
worin der Vorgang des Erkennens der fibro-glandulären Geweberegion im Ultraschallbild umfasst:
einen Vorgang des Erzeugens einer fibro-glandulären Inferenzregionsmaske im Ultraschallbild unter Verwendung eines Deep-Learning-Modelles;
einen Vorgang des Erzeugens einer fibro-glandulären Regionsbinärkarte aufgrund der erzeugten fibro-glandulären Inferenzregionsmaske; und
einen Vorgang des Erzeugens einer fibro-glandulären Regionsteilungsmaske (21) aufgrund der fibro-glandulären Regionsbinärkarte.

12. Rechenvorrichtung umfassend:
zumindest einen Prozessor; und
einen Speicher,
worin der zumindest eine Prozessor dazu konfiguriert ist, um:
ein Ultraschallbild (S10, S110) zu erfassen,
eine Brustregion im Ultraschallbild (S11, S120) zu erkennen,
eine fibro-glanduläre Geweberegion im Ultraschallbild zu erkennen, umfassend Erzeugen zumindest einer von einer fibro-glandulären Inferenzregionsmaske und einer fibro-glandulären Regionsbinärkarte unter Verwendung eines Deep-Learning-Modelles (S12, S130); und
zumindest einen von einem Brustgewebeanteil (S14) oder einem Drüsengewebeanteil (S15) aufgrund der erkannten Brustregion und der erkannten fibro-glandulären Geweberegion zu quantifizieren, umfassend Durchführen von Histogramm-basierter Normalisierung innerhalb einer brustinternen Interessenregion, um ein Drüsengewebeanteilverhältnis (S140) zu bestimmen,
**dadurch gekennzeichnet, dass** das Erkennen der Brustregion umfasst:
Erzeugen einer Brustinferenzregionsmaske im Ultraschallbild unter Verwendung eines Deep-Learning-Modelles,
Erzeugen einer Brustregionsbinärkarte aufgrund der erzeugten Brustinferenzregionsmaske, und
Erzeugen einer Brustregionsteilungsmaske aufgrund einer größten Region unter den Regionen, die in der Brustregionsbinärkarte umfasst sind,
worin das Erkennen der fibro-glandulären Geweberegion im Ultraschallbild umfasst:
Erzeugen einer fibro-glandulären Inferenzregionsmaske im Ultraschallbild unter Verwendung eines Deep-Learning-Modelles;
Erzeugen einer fibro-glandulären Regionsbinärkarte aufgrund der erzeugten fibro-glandulären Inferenzregionsmaske; und
Erzeugen einer fibro-glandulären Regionsteilungsmaske (21) aufgrund der fibro-glandulären Regionsbinärkarte.

## Revendications

1. Procédé de quantification d'une composition mammaire réalisé par un dispositif informatique, le procédé comprenant :
l'acquisition d'une image échographique (S10, S110) ;
l'identification d'une région mammaire dans l'image échographique (S11, S120) ;
l'identification d'une région de tissu fibroglandulaire dans l'image échographique, comprenant la génération d'au moins l'un parmi un masque de région d'inférence fibroglandulaire et une carte binaire de région fibroglandulaire à l'aide d'un modèle d'apprentissage profond (S12, S130) ; et
la quantification d'au moins l'un parmi un composant de tissu mammaire (S14) ou un composant de tissu glandulaire (S15) sur la base de la région mammaire identifiée et de la région de tissu fibroglandulaire identifiée, comprenant la réalisation d'une normalisation basée sur histogramme dans une région intramammaire d'intérêt pour déterminer un rapport de composant de tissu glandulaire (S140),
**caractérisé en ce que** l'identification de la région mammaire comprend :
la génération d'un masque de région d'inférence mammaire dans l'image échographique à l'aide d'un modèle d'apprentissage profond, la génération d'une carte binaire de région mammaire sur la base du masque de région d'inférence mammaire généré, et la génération d'un masque de division de région mammaire (11) sur la base d'une région la plus grande parmi des régions incluses dans la carte binaire de région mammaire,
dans lequel l'identification de la région de tissu fibroglandulaire dans l'image échographique comprend :
la génération d'un masque de région d'inférence fibroglandulaire dans l'image échographique à l'aide d'un modèle d'apprentissage profond ;
la génération d'une carte binaire de région fibroglandulaire sur la base du masque de région d'inférence fibroglandulaire généré ; et
la génération d'un masque de division de région fibroglandulaire (21) sur la base de la carte binaire de région fibroglandulaire.

2. Procédé selon la revendication 1, dans lequel le masque de région d'inférence mammaire comprend une carte de probabilité de région mammaire, et
dans lequel la génération de la carte binaire de région mammaire sur la base du masque de région d'inférence mammaire généré comprend :
la génération de la carte binaire de la région mammaire en classant la carte de probabilité de région mammaire sur la base d'un seuil prédéterminé.

3. Procédé selon la revendication 1, dans lequel le masque de région d'inférence fibroglandulaire comprend une carte de probabilité de région fibroglandulaire, et
dans lequel la génération de la carte binaire de région fibro-glandulaire sur la base du masque de région d'inférence fibro-glandulaire généré comprend :
la génération de la carte binaire de région fibroglandulaire en classant la carte de probabilité de région fibroglandulaire sur la base d'un seuil prédéterminé.

4. Procédé selon la revendication 1, dans lequel la génération du masque de division de région fibroglandulaire (21) sur la base de la carte binaire de région fibroglandulaire comprend :
la génération d'un masque de région fibroglandulaire intramammaire en réalisant une opération pour le masque de division de région mammaire (11) et la carte binaire de région fibroglandulaire ; et
la génération du masque de division de région fibroglandulaire (21) sur la base du masque de région fibroglandulaire intramammaire.

5. Procédé selon la revendication 4, dans lequel le masque de division de région fibroglandulaire est acquis sur la base d'une région la plus grande parmi des régions incluses dans le masque de région fibroglandulaire intramammaire.

6. Procédé selon la revendication 1, dans lequel la quantification d'au moins l'un parmi le composant de tissu mammaire (S14) ou le composant de tissu glandulaire (S15) sur la base de la région mammaire identifiée et de la région de tissu fibroglandulaire identifiée (S140) comprend :
l'identification d'une région de tissu glandulaire sur la base de la région de tissu fibroglandulaire identifiée ; et
la quantification du composant de tissu glandulaire en analysant la région de tissu fibroglandulaire identifiée et la région de tissu glandulaire identifiée.

7. Procédé selon la revendication 6, dans lequel l'identification de la région de tissu glandulaire sur la base de la région de tissu fibroglandulaire identifiée comprend :
la génération d'une carte binaire de région de tissu glandulaire sur la base d'un masque de division de région fibroglandulaire, et
la génération d'un masque de division de la région tissulaire glandulaire (31) sur la base de la carte binaire de région de tissu glandulaire.

8. Procédé selon la revendication 7, dans lequel la génération de la carte binaire de région de tissu glandulaire sur la base du masque de division de région fibro-glandulaire comprend :
l'extraction d'un masque de division de région de tissu glandulaire d'intérêt à partir du masque de division de région fibro-glandulaire ;
la détection d'une région d'intérêt de type polygonal comprenant la région de tissu glandulaire d'intérêt ;
la réalisation d'une normalisation basée sur histogramme pour une image échographique à l'intérieur de la région d'intérêt de type polygonal ;
la génération d'une image échographique de normalisation de région d'intérêt en utilisant l'image échographique à l'intérieur de la région d'intérêt de type polygonal dans laquelle la normalisation basée sur histogramme est réalisée et une image échographique à l'extérieur de la région d'intérêt de type polygonal ;
la génération d'une image de région de tissu glandulaire d'intérêt en réalisant une opération pour l'image échographique de normalisation de région d'intérêt et le masque de région de tissu glandulaire d'intérêt ; et
la génération de la carte binaire de région de tissu glandulaire en classant l'image de région de tissu glandulaire d'intérêt sur la base d'un seuil prédéterminé.

9. Procédé selon la revendication 1, dans lequel la quantification d'au moins l'un parmi le composant de tissu mammaire (S14) ou le composant de tissu glandulaire (S15) sur la base de la région mammaire identifiée et de la région de tissu fibroglandulaire identifiée (S140) comprend :
le calcul d'un rapport du composant de tissu mammaire en utilisant une valeur de pixel d'un masque de division de région fibroglandulaire et une valeur de pixel d'un masque de division de région mammaire, et
le mappage du rapport du composant de tissu mammaire en fonction d'un grade de composant de tissu mammaire (BTC).

10. Procédé selon la revendication 1, dans lequel la quantification d'au moins
l'un parmi le composant de tissu mammaire (S14) ou le composant de tissu glandulaire (S15) sur la base de la région mammaire identifiée et de la région de tissu fibroglandulaire identifiée (S140) comprend :
le calcul d'un rapport du composant de tissu glandulaire en utilisant une valeur de pixel d'un masque de division de région de tissu glandulaire (31) et une valeur de pixel d'un masque de division de région fibroglandulaire (21), et
le mappage du rapport du composant de tissu glandulaire selon un grade de composant de tissu glandulaire (GTC).

11. Support de stockage non transitoire lisible par ordinateur comprenant un programme informatique, dans lequel lorsque le programme informatique est exécuté par un ou plusieurs processeurs, le programme informatique permet aux un ou plusieurs processeurs de réaliser les opérations suivantes pour quantifier une composition mammaire, les opérations comprenant :
une opération d'acquisition d'une image échographique (S10, S110) ;
une opération d'identification d'une région mammaire dans l'image échographique (S11, S120) ;
une opération d'identification d'une région de tissu fibroglandulaire dans l'image échographique, comprenant la génération d'au moins un masque de région d'inférence fibroglandulaire et d'une carte binaire de région fibroglandulaire à l'aide d'un modèle d'apprentissage profond (S12, S130) ; et
une opération de quantification d'au moins l'un parmi un composant de tissu mammaire (S14) ou un composant de tissu glandulaire (S15) sur la base de la région mammaire identifiée et de la région de tissu fibroglandulaire (S140) identifiée, comprenant la réalisation d'une normalisation basée sur histogramme dans une région intramammaire d'intérêt pour déterminer un rapport de composant de tissu glandulaire (S140),
**caractérisé en ce que** l'opération d'identification de la région mammaire comprend :
une opération de génération d'un masque de région d'inférence mammaire dans l'image échographique à l'aide d'un modèle d'apprentissage profond ,
une opération de génération d'une carte binaire de région mammaire sur la base du masque de région d'inférence mammaire généré, et
une opération de génération d'un masque de division de région mammaire sur la base d'une région la plus grande parmi des régions incluses dans la carte binaire de région mammaire,
dans lequel l'opération d'identification de la région de tissu fibroglandulaire dans l'image échographique comprend :
une opération de génération d'un masque de région d'inférence fibroglandulaire dans l'image échographique à l'aide d'un modèle d'apprentissage profond ;
une opération de génération d'une carte binaire de région fibroglandulaire sur la base du masque de région d'inférence fibroglandulaire généré ; et
une opération de génération d'un masque de division de région fibroglandulaire (21) sur la base de la carte binaire de région fibroglandulaire.

12. Dispositif informatique comprenant :
au moins un processeur ; et
une mémoire,
dans lequel l'au moins un processeur est configuré pour :
acquérir une image échographique (S10, S110),
identifier une région mammaire dans l'image échographique (S11, S120),
identifier une région de tissu fibroglandulaire dans l'image échographique,
comprenant la génération d'au moins l'un parmi un masque de région d'inférence fibroglandulaire et une carte binaire de région fibroglandulaire à l'aide d'un modèle d'apprentissage profond (S12, S130) ; et
quantifier au moins l'un parmi un composant de tissu mammaire (S14) ou un composant de tissu glandulaire (S15) sur la base de la région mammaire identifiée et de la région de tissu fibroglandulaire identifiée, comprenant la réalisation d'une normalisation basée sur histogramme dans une région intramammaire d'intérêt pour déterminer un rapport de composant de tissu glandulaire (S140),
**caractérisé en ce que** l'identification de la région mammaire comprend :
la génération d'un masque de région d'inférence mammaire dans l'image échographique à l'aide d'un modèle d'apprentissage profond ,
la génération d'une carte binaire de région mammaire sur la base du masque de région d'inférence mammaire généré , et
la génération d'un masque de division de région mammaire sur la base d'une région la plus grande parmi des régions incluses dans la carte binaire de région mammaire,
dans lequel l'identification de la région de tissu fibroglandulaire dans l'image échographique comprend :
la génération d'un masque de région d'inférence fibroglandulaire dans l'image échographique à l'aide d'un modèle d'apprentissage profond ;
la génération d'une carte binaire de région fibroglandulaire sur la base du masque de région d'inférence fibroglandulaire généré ; et
la génération d'un masque de division de région fibroglandulaire (21) sur la base de la carte binaire de région fibroglandulaire.
